**Europäisches Patentamt**

**European Patent Office**

**Office européen des brevets**

(19)

(11) Veröffentlichungsnummer: **0 049 849**
**B1**

(12) # EUROPÄISCHE PATENTSCHRIFT

(45) Veröffentlichungstag der Patentschrift:
05.12.84

(21) Anmeldenummer: 81107962.3

(22) Anmeldetag: 06.10.81

(51) Int. Cl.³: **C 12 Q 1/34**, G 01 N 33/54

(54) Verfahren zur Bestimmung der Streptokokken-Desoxiribonuclease B nach der Toluidinblau O-Methode.

(30) Priorität: 10.10.80 DE 3038286

(43) Veröffentlichungstag der Anmeldung:
21.04.82 Patentblatt 82/16

(45) Bekanntmachung des Hinweises auf die Patenterteilung:
05.12.84 Patentblatt 84/49

(84) Benannte Vertragsstaaten:
BE DE FR IT SE

(56) Entgegenhaltungen:
EP - A - 0 017 909
FR - A - 2 242 685

CHEMICAL ABSTRACTS; Band 85, Nr. 25, 20. Dezember 1976, Seite 182, Nr. 188207e, Columbus, Ohio, U.S.A., V.I. KOCHEROVETS: "Modification of a qualitative method for determining deoxyribonuclease activity in staphylococci"

(73) Patentinhaber: **BEHRINGWERKE AKTIENGESELLSCHAFT, Postfach 1140, D-3550 Marburg/Lahn (DE)**

(72) Erfinder: **Gils, Reiner, Am Winterbaum 2, D-3552 Wetter (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Beschreibung

Die Erfindung betrifft ein Verfahren zur Bestimmung der Desoxiribonuclease B sowie Antikörper, die gegen Desoxiribonuclease B gerichtet sind, mittels eines Toluidinblau O-Desoxiribonucleinsäure-Komplexes.

Desoxiribonucleasen (DNasen) sind extrazelluläre Stoffwechselprodukte von Streptokokken der Gruppe A nach Lancefield. Serologisch und elektrophoretisch lassen sich vier DNasen unterscheiden, die mit A, B, C und D bezeichnet werden. Diese induzieren im Menschen die Bildung spezifischer Antikörper. Für die Erkennung von Streptokokkeninfektionen hat die Bestimmung der DNase B-Antikörper diagnostische Bedeutung erlangt, da dieses Enzym von Streptokokken der Gruppe A vorwiegend gebildet wird.

Bei dem von Streptokokken hervorgerufenen rheumatischen Fieber stellt man neben einem Anstieg des Anti-Streptolysin-Titers häufig auch einen Anti-DNase B-Anstieg fest.

Zumeist besteht zwischen diesen Werten eine gute Übereinstimmung, darüber hinaus bietet die Anti-DNase B-Bestimmung einige zusätzliche diagnostische Vorteile. Vor allem bei Hautinfektionen kommt eine Erhöhung des Anti-Streptolysin-Gehaltes sehr selten vor, während ein kräftiger Anstieg des ADNase B-Titers beobachtet wird. Es besteht daher ein Bedürfnis für die Bestimmung der Anti-Streptokokken-DNase B.

Aus der deutschen Patentschrift 23 44 441 ist ein Verfahren zur Bestimmung von Streptokokken-Desoxiribonuclease B bekannt, in welchem ein Komplex aus dem metachromatischen Farbstoff Toluidinblau O und einer Desoxiribonucleinsäure als Substrat verwendet wird. Beim enzymatischen Abbau dieses Substrates fällt der Farbstoff flockig aus, und die überstehende Lösung entfärbt sich.

Ein Nachteil dieses beschriebenen Verfahrens ist die lange Inkubationsdauer für die Enzymreaktion.

Es wurde nun ein Verfahren zur Bestimmung von Streptokokken-DNase B oder ihrer Antikörper auf der Grundlage des Toluidinblau O-Verfahrens gefunden, dessen wesentlicher Vorteil in seiner schnelleren Durchführbarkeit besteht.

Gegenstand der Erfindung ist ein Verfahren zur Bestimmung der DNase B, indem man einer Reihenverdünnung einer Lösung von DNase B oder einer Lösung von Anti-DNase B, der zuvor eine definierte überschüssige Menge DNase B zugesetzt wurde, einen DNA-Toluidinblau O-Komplex zusetzt und die Fällungsgrenzkonzentration bestimmt, dadurch gekennzeichnet, dass nach Zugabe des Komplexes eine Metallhydroxidlösung als Fällungsmittel zugesetzt wird.

Dem Fällungsmittel ist gegebenenfalls eine Aminosäure und/oder ein Salz zugesetzt.

Es ist überraschenderweise gefunden worden, dass eine sichtbare Präzipitation des freien Farbstoffes längere Zeit in Anspruch nimmt als die Enzym-Substrat-Reaktion, die Präzipitation durch Alkalisierung des Testansatzes jedoch wesentlich beschleunigt werden kann.

Wegen der einfachen Handhabung eignen sich aus der Gruppe der anorganischen Basen vor allem wässerige Lösungen von Erdalkali- oder Alkalihydroxiden, vor allem Calcium-, Kalium- oder Natriumhydroxidlösungen.

Bevorzugt ist eine Metallhydroxidlösung mit Aminosäure- oder Salzzusatz.

Geeignete Aminosäuren sind beispielsweise Glycin, β-Alanin, Leucin, Sarkosin, Alanin oder Glutaminsäure.

Geeignete Salze sind beispielsweise Natrium-, Kalium- oder Magnesiumchlorid, Kalium- oder Magnesiumsulfat oder allgemein Chloride und Sulfate von Alkali- und Erdalkalimetallen.

Als erfindungsgemässes Fällungsmittel eignet sich insbesondere ein Gemisch aus folgenden Bestandteilen:

Zu einer 0,5 bis 5 molaren Erdalkali- oder Alkalihydroxidlösung, vorzugsweise 2 bis 4 molar, setzt man 1 bis 10%, vorzugsweise 5%, oder in molarer Konzentration Aminosäure oder Salz zu. Die Vermischung der beiden, gegebenenfalls auch mehreren Komponenten erfolgt in wässriger Lösung bei 10 bis 100 °C, vorzugsweise bei Raumtemperatur. Als Fällungsmittel kann auch eine 0,5 bis 5 molare basische Lösung ohne Zusatz von Aminosäure oder Salz dienen; In diesem Fall empfiehlt es sich, die Reaktion nicht später als 30 Minuten nach Zusatz des Fällungsmittels zu dem Testansatz abzulesen.

Zur Herstellung eines geeigneten Fällungsmittels kann wie folgt vorgegangen werden: 20 bis 200 Gramm Natriumhydroxid in 1000 ml destilliertem Wasser werden unter ständigem Rühren bei 10 bis 100 °C 10 bis 100 Gramm Natriumchlorid zugesetzt. Nach vollständigem Lösen der beiden Komponenten wird die Lösung filtriert.

Gegenstand der Erfindung ist ferner ein Verfahren zur Ausfällung des freien Farbstoffes nach einer Inkubationsdauer von 20 bis 60 Minuten, vorzugsweise nach 30 bis 40 Minuten, und anschliessender kurzer Erwärmung des Testansatzes auf 40 bis 70 °C, vorzugsweise 50 bis 60 °C, für eine Dauer von 2 bis 15 Minuten, vorzugsweise 5 bis 10 Minuten, zur Aggregierung der entstandenen Präzipitate.

Das erfindungsgemässe Verfahren ist wegen seiner Einfachheit für Serieanalysen geeignet.

Zweckmässig ist die Zusammenstellung eines sogenannten Testkits, der sich aus folgenden Reagenzien zusammensetzt:

1. DNA-Toluidinblau O-Substrat

Das Substrat wird nach bekannten Verfahren hergestellt (DE-PS 23 44 441).

2. Streptokokken-DNase B, z.B. eine Enzympräparation aus dem Kulturfiltrat von Streptokokken der Gruppe A, hergestellt nach L.W. Wannamaker und W. Yasmineh, J. exp. Med. 126 (1967) 475. Von Vorteil ist der Zusatz von Erdalkaliionen, vorzugsweise von $Mg^{++}$-Ionen, in 0,001 bis 0,5 molarer Konzentration, vorzugsweise 0,001 bis 0,2 molar, zur Stabilisierung der Enzymaktivität.

Die Lösung ist mit einer Aktivität von ca. 30 U/ml nach Kunitz gebrauchsfertig.

3. Imidiazol-Pufferlösung pH 7,8.

Der Anti-DNase B-Puffer ist ein Imidazol-Puffer pH 7,8 (17,0 g Imidazol/l), der 2,0 Gramm Serumalbumin vom Rind, 1,47 Gramm $CaCl_2 \times 2H_2O$ und 0,6 Gramm $MgSO_4 \times 7H_2O$ im Liter enthält. Die Pufferlösung, die ausserdem Natriumazid (1,0 g/l) enthält, wird zum Herstellen der Serum-Verdünnungsreihen verwendet.

4. Fällungsmittel-Reagenz, bestehend aus einer wässrigen Hydroxidlösung mit Stabilisatorzusatz. In der bevorzugten Form ist dies 2,5 M NaOH (100 g/l) und 1 M NaCl (58,5 g/l).

5. Anti-Streptokokken-DNase B-Standard-Serum ist ein Humanserum mit einer definierten Konzentration von Anti-Streptokokken-DNase B, das bei vorschriftsmässiger Durchführung des Testes einen Anti-DNase B-Titer von 300 ergibt.

Das Verfahren wird bevorzugt in Reihenanalysen durchgeführt, wobei Verdünnungsreihen der Lösungen, in denen die Konzentration der Anti-DNase B bestimmt werden soll, angelegt werden. Wird Serum verwendet, ist es zweckmässig, dieses kurzzeitig auf etwa +56 °C zu erwärmen. Dabei werden unspezifische Reaktionen, die das Messergebnis verfälschen könnten, ausgeschaltet. Zu jeder Verdünnung der Probe wird eine bekannte Menge der DNase B-Lösung gegeben, die man mit der Anti-DNase B reagieren lässt. Danach wird die Probe mit DNA-Toluidinblau-Substrat versetzt, auf welches nur die nicht durch Anti-DNase B gebundene DNase B einwirken kann. Bei dem enzymatischen Abbau des DNA-Toluidinblau O-Komplexes wird der Farbstoff frei, der durch Zugabe des Fällungsmittels präzipitiert wird. Die jeweiligen Verdünnungsreihen werden mit Standardreihen der Anti-DNase B in Vergleich gesetzt.

Für die Durchführung des Testes ist eine Mikrotiterplatte besonders geeignet, da hierauf neben einer Reihe von Sera eine Standardreihe für die Anti-DNase B sowie entsprechende Kontrollen für das Enzym und das Substrat nebeneinander aufgetragen werden können.

Das folgende Beispiel erläutert die Erfindung:

1. Vorbereitung der Reagenzien:

1.1. DNA-Toluidinblau O-Substrat, lyophilisiert, wird in 5 ml destilliertem Wasser aufgenommen.

1.2. Streptokokken-DNase, lyophilisiert, in 3 ml destilliertem Wasser aufnehmen.

1.3. Fällungsmittel-Reagenz (Toluidinblau O-Fällungsmittel) ist eine wässrige Lösung von 0,5 g Natriumhydroxid und 0,29 g Natriumchlorid in 5 ml Wasser.

1.4. Anti-Streptokokken-DNase B-Standard-Serum ist ein flüssiges, stabilisiertes Humanserum, das bei vorschriftsmässiger Testdurchführung einen Anti-DNase B-Titer von 300 besitzt. Das Standard-Serum ist gebrauchsfertig.

1.5. Imidazol-Pufferlösung pH 7,8 ist gebrauchsfertig.

2. Herstellung der Serum-Verdünnungsreihen:

2.1. Jedes zu untersuchende Serum wird 30 Minuten in einem Wasserbad bei +56 °C inaktiviert, hierfür wird eine 1:50 Verdünnung des Patientenserums verwendet.

Röhrchen A: 0,1 ml Serum +4,9 ml Imidazol-Pufferlösung pH 7,8 (Serumverdünnung 1:50) 2.2. Nach Inaktivierung aus Röhrchen A eine zweite Serumverdünnung 1:75 herstellen.

Röhrchen B: 1,0 ml Serumverdünnung A +0,5 ml Imidazol-Pufferlösung pH 7,8 (Serumverdünnung 1:75).

2.3. Verdünnungsstufen A (1:50) und B (1:75) ebenso vom Anti-DNase B-Standard-Serum herstellen, welches nicht inaktiviert zu werden braucht.

2.4. In einer Mikrotiterplatte Verdünnungsreihen von Standard- und Patientensera von 1:50 bis 1:2400 mit einem Volumen von 25 µl herstellen.

3. Testansatz:

3.1. Jeder Vertiefung 25 µl der gebrauchsfertigen Streptokokken-DNase B-Lösung zugeben, mischen, Platte verschliessen und 15 Minuten bei Raumtemperatur stehenlassen.

3.2. Anschliessend in alle Vertiefungen 50 µl DNA-Toluidinblau O-Substrat mit einer Mikropipette einfüllen, mischen, die Platte verschliessen und 30 Minuten in einem Brutschrank bei +37 °C inkubieren.

3.3. In allen Vertiefungen 25 µl basisches Fällungsmittel einfüllen, gut mischen, Platte verschliessen und 5–10 Minuten bei +56 °C im Brutschrank oder Wasserbad stehenlassen.

4. Bewertung:

Zum Ablesen Platte auf eine Milchglasscheibe, die von unten beleuchtet werden kann, oder auf einen Ablesespiegel stellen. Die Resultate können auch sehr gut abgelesen werden, wenn die Platte gegen eine weisse Fläche gehalten wird. Es werden diejenigen Verdünnungsstufen bei Standard- und Patientensera notiert, bei denen eine rötlichviolette Präzipitation noch nicht wahrnehmbar ist, und daraus die Anti-Streptokokken-DNase B-Konzentration im Patientenserum berechnet:

Multiplikation von Verdünnungsfaktor des Patientenserums mit Anti-DNase B-Konzentration im Standard-Serum (Titerstufe 300) dividiert durch Verdünnungsfaktor im Standard-Serum.

Z. B. Standard-Serum: homogene rötliche Lösung Vertiefung Nr. 5 (1:200)

Patientenserum: homogene rötliche Lösung Vertiefung Nr. 7 (1:400)

ergibt

$$\frac{400 \times 300}{200} = 600 \text{ (Titer)}$$

Durch das erfindungsgemässe Verfahren lässt sich gegenüber dem herkömmlichen Toluidinblau O-Verfahren eine wesentliche Zeitersparnis und eine verbesserte Ablesbarkeit erzielen. Bei dem herkömmlichen Verfahren beträgt die Inkubationsdauer für die Enzymreaktion 4 Stunden, bei dem erfindungsgemässen Verfahren 30 Minuten. Besteht bei dem herkömmlichen Verfahren keine Zentrifugiermöglichkeit, müssen die Platten in der Regel weitere 2 Stunden zur Verbesserung der Ablesbarkeit stehengelassen werden. Diese Standzeit der Platten entfällt bei dem neuen Verfahren ebenfalls. Insgesamt lässt sich durch das erfindungsgemässe Verfahren eine Zeitersparnis von ca. 5 Stunden erzielen, Zentrifugieren der Platten zur Erzielung einer besseren Ablesbarkeit ist ebenfalls nicht nötig.

**Patentansprüche**

1. Verfahren zur Bestimmung der Desoxiribonuclease B, indem man einer Reihenverdünnung einer Lösung von Desoxiribonuclease B oder einer Lösung von Anti-Desoxiribonuclease B, der zuvor eine definierte überschüssige Menge Desoxiribonuclease B zugesetzt wurde, einen DNA-Toluidinblau O-Komplex zusetzt und die Fällungsgrenzkonzentration bestimmt, dadurch gekennzeichnet, dass nach Zugabe des Komplexes eine Metallhydroxidlösung zugesetzt wird.

2. Verfahren nach Anspruch 1, dadurch gekennzeichnet, dass die Metallhydroxidlösung eine Aminosäure und/oder ein Salz enthält.

3. Verfahren nach Anspruch 1, dadurch gekennzeichnet, dass die Metallhydroxidlösung eine Lösung mit 2 bis 20% eines Alkalimetallhydroxids ist, die gegebenenfalls 0 bis 10% eines Salzes enthält.

4. Verfahren nach Anspruch 1, dadurch gekennzeichnet, dass die Metallhydroxidlösung, die gegebenenfalls eine Aminosäure und/oder ein Salz enthält, dem Reaktionsansatz 15 bis 60 Minuten nach Start der Enzymreaktion zugesetzt und der Reaktionsansatz anschliessend für 2 bis 15 Minuten auf 40 bis 70 °C erwärmt wird.

**Revendications**

1. Procédé de dosage de la désoxyribonucléase B, dans lequel on ajoute à une dilution en série d'une solution de désoxyribonucléase B ou d'une solution d'anti-désoxyribonucléase B à laquelle on a ajouté au préalable un excès déterminé de désoxyribonucléase B, un complexe DNA-bleu de toluidine O et on détermine la concentration limite de précipitation, caractérisé en ce qu'aprés l'addition du complexe, on ajoute une solution d'hydroxyde métallique.

2. Procédé suivant la revendication 1, caractérisé en ce que la solution d'hydroxyde métallique contient un aminoacide et/ou un sel.

3. Procédé suivant la revendication 1, caractérisé en ce que la solution d'hydroxyde métallique est une solution avec 2 à 20% d'un hydroxyde de métal alcalin qui contient le cas échéant 0 à 10% d'un sel.

4. Procédé suivant la revendication 1, caractérisé en ce que la solution d'hydroxyde métallique, qui contient le cas échéant un aminoacide et/ou un sel, est ajoutée au mélange réactionnel 15 à 60 minutes après le démarrage de la réaction enzymatique et en ce que le mélange réactionnel est ensuite chauffé pendant 2 à 15 minutes à 40 à 70 °C.

**Claims**

1. A process for determining the desoxyribonuclease B by adding a DNA/toluidine blue O complex to a serial dilution of desoxyribonuclease B or of a solution of antidesoxyribonuclease B to which a defined excess quantity of desoxyribonuclease B has previously been added, and determining the limiting concentration for precipitations, wherein a metal hydroxide solution is added after the addition of the complex.

2. A process as claimed in claim 1, wherein the metal hydroxide solution contains an amino acid and/or a salt.

3. A process a claimed in claim 1, wherein the metal hydroxide solution is a solution of from 2 to 20% of an alkali metal hydroxide which optionally contains 0 to 10% of a salt.

4. A process as claimed in claim 1, wherein the metal hydroxide solution, which optionally contains an amino acid and/or a salt, is added to the reaction mixture 15 to 60 minutes after start of the enzymatic reaction and the reaction mixture is then warmed to 40 to 70 °C for 2 to 15 minutes.